# EUROPEAN PATENT APPLICATION

(11) **EP 0 937 481 A1**
(43) Date of publication of application: **25.08.1999**
(21) Application number: 99300939.8
(22) Date of filing: 09.02.1999
(51) Int. Cl.: A61M 25/00

(54) **Catheter or guidewire with varying flexibility**

(30) Priority: 19.02.1998 US 25912
(71) Applicant: Precision Vascular Systems, Inc., Salt Lake City, Utah 84108 (US)
(72) Inventor: Jacobsen, Stephen C., Salt Lake City, Utah 84102 (US); Lippert, John, Park City, Utah 84098 (US)
(74) Representative: Gilmour, David Cedric Franklyn

(57) **Abstract**

A catheder/guidewire for threading into a vasculature passageway includes an elongate body dimensioned for threading into the passageway, where the body has one or more sections intermediate the proximal end and distal end, further to be more flexible than other intermediate sections so that the more flexible sections coincide with curves in the passageway when the elongate body is threaded into the passageway.

The variation in flexibility is realized by radially cutted slots. Number of cuts per length unit, depth and width of slots determine the local stiffness.

## Description

### BACKGROUND OF THE INVENTION

This invention relates to a catheter/guidewire device along which st!ected locations are made especially flexible to coincide generally with curves of a pathway profile of a body vasculature into which the device is to be inserted.

Catheters have become an indispensable tool in diagnosing and treating various disorders in the human body. Since catheters can be threaded (typically over a catheter guidewire) through tortuous vasculature passageways to a target locations, it is possible to access the target location for restoring blood flow, viewing, testing, occluding, delivery of medicaments, etc. as desired.

Navigation through the anatomy is typically achieved by viewing a guidewire (having a radiopaque element) in the body using X-ray fluoroscopy. The guidewire is inserted into a vessel or duct (along with the catheter if desired) and moved therethrough until the guidewire tip reaches the desired location. Of course, during insertion of the guidewire, it may be necessary to rotate the proximal end to direct the typically curved tip thereof into a desired vessel or duct branch, and then advance the guidewire further. The catheter is threaded over the guidewire to follow or track the wire to the desired location, and provide additional support for the wire. Once the catheter is in place, the guidewire may be withdrawn, depending upon the therapy to be performed.

As the guidewire is advanced into the anatomy, internal resistance from the typically numerous turns and curves and surface contact decreases the ability to advance the guidewire further. This, in turn, may lead to a more difficult and prolonged procedure, or more seriously, failure to access the desired anatomy and thus a failed procedure. A guidewire and/or catheter with both flexibility at appropriate locations and good torque characteristics (torsional stiffness) would, of course, help overcome the problems created by the internal resistance. Also, once the catheter were in place, if its flexibility better accommodated the turns and curves of the passageway in which it was inserted, less trauma would result to the passageway.

### SUMMARY OF THE INVENTION

It is an object of the invention to provide a catheter and/or catheter guidewire in which one or more sections intermediate the proximal end the distal end are formed to be more flexible than other intermediate sections.

It is also an object of the invention to provide such a catheter and/or catheter guidewire in which some of the more flexible sections are closer to the proximal end.

It is a further object of the invention to provide such a catheter and/or catheter guidewire wherein the more flexible sections coincide with the more curved portions of a vasculature passageway into which the catheter and/or catheter guidewire is to be inserted.

It is still another object of the invention, in accordance with one aspect thereof, to provide such a catheter and/or catheter guidewire in which the more flexible sections are formed with a plurality of generally transverse cuts spaced-apart longitudinally in the sections.

The above and other objects of the invention are realized in a specific illustrative embodiment of a catheter/guidewire adapted for threading into a vasculature passageway having a determinable pathway profile of curves and generally linear sections. The catheter/guidewire comprises an elongate body having a distal end, a proximal end and intermediate sections, with selected intermediate sections being formed to be more flexible than other intermediate sections so that the selected sections coincide with the curves of the pathway profile when the device is threaded into the vasculature passageway.

In accordance with one aspect of the invention, the selected more flexible sections are formed with a plurality of generally transverse cuts either made deeper, wider, or closer together to increase flexibility.

### BRIEF DESCRIPTION OF THE DRAWINGS

The above and other objects, features and advantages ofthe invention will become apparent from a consideration of the following detailed description presented in connection either accompanying drawings in which:
FIG. 1 is a side, cross-sectional view of a catheter and/or catheter guidewire disposed in a vasculature passageway after having been constructed in accordance with the principles of the present invention;
FIG. 2 is a stiffness/flexibility per distance graph of the catheter and/or catheter guidewire of FIG. 1; and
FIG. 3 is a side, fragmented, cross-sectional view of a catheter and/or catheter guidewire showing various types of cuts which may be employed to achieve desired flexibility.

### DETAILED DESCRIPTION

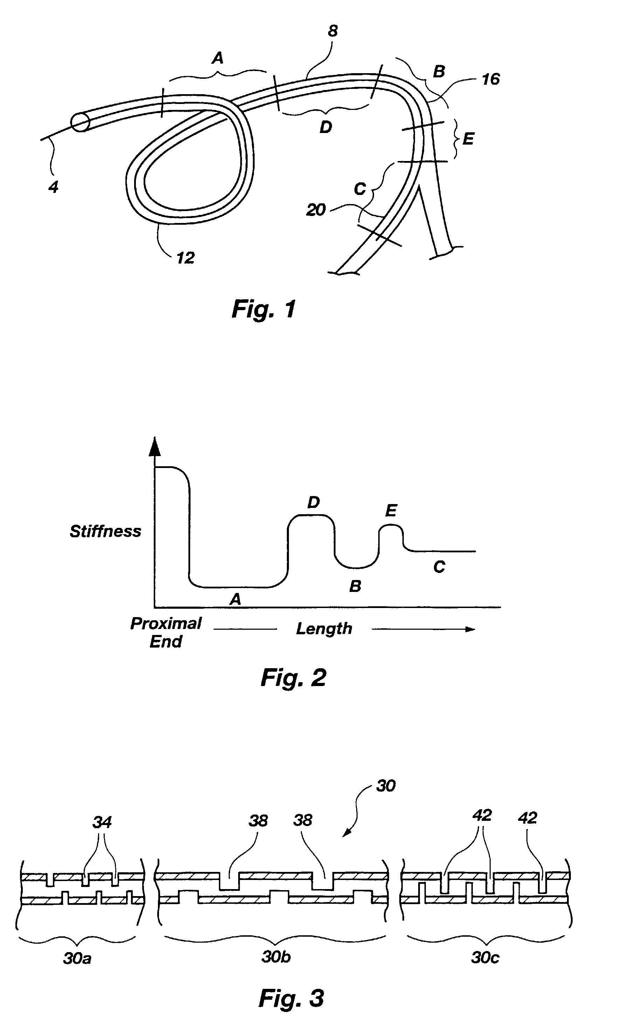

Referring to FIG. 1, there is shown a side, cross-sectional view of a catheter or catheter guidewire 4 threaded into a vasculature passageway 8. The vasculature passageway 8 is shown with a first sharp curve 12 which actually loops, a second less sharp but still severe curve 16, and a third more gradual curve 20 (of the branch to be selected). These curved sections of the vasculature passageway are also labeled A, B and C respectively.

For the vasculature passageway 8 to better accommodate the catheter or catheter guidewire device 4, the device is formed with more flexible sections at locations along the length of the device which coincide with the locations of the curves A, B and C. In particular, that portion of the device 4 which would lie or reside in the curved portion A of the vasculature passageway 8 when the device were threaded into the passageway is constructed to be most flexible whereas that portion of the device lying or residing in curved portion B of the passageway 8 is constructed to be next most flexible, and that part of the device residing in curved portion C of the passageway 8 is made to be flexible but the least flexible of the three sections (unless it also happens to coincide with the distal end of the device 4 in which case portion C would also be very flexible for navigation purposes) Portions A, B and C should be somewhat longer than curved portions of the vasculature passageway 8 they occupy so as to avoid tending to "lock" the device 4 in place, once inserted. In this manner, the catheter or catheter guidewire device 4 can be threaded into the vasculature passageway 8 and effectively "fit" comfortably in the curved profile of the passageway.

FIG. 2 is a graph where stiffness/flexibility is plotted against the length of the catheter or catheter/guidewire device 4 of FIG. 1 extending from the proximal end to the distal end. In particular, as the graph shows, Section A (shown in FIG. 1) of the device 4 is the most flexible section, on either side of which are sections of much greater stiffness; Section B is the next most flexible, again with either side of the section being much stiffer; and Section C is the least flexible of the three identified sections. Section C, of course, is at the distal end of the device 4 whereas Section A is near the proximal end and Section B is intermediate the two.

FIG. 2 shows the relative stiffness/flexibility of various segments of the device 4 so that it is readily accommodated in the vasculature passageway 8 of FIG. 1. Of course, for other pathway profiles for passageways, different stiffness/flexibility characteristics would be provided for whatever catheter or catheter guidewire were to be threaded into that passageway. One simple configuration, suitable for use in conjunction with a carotid syphon, for example, is a catheter or catheter guidewire which is more flexible at a proximal segment than at the distal end, where the distal end may have varying lengths relative to the flexible location at the proximal segment.

It will be noted that the most flexible section of the catheter or catheter guidewire device 4 is not at or just at the distal end as is the case with catheter or catheter/guidewire devices. Rather, the stiffness or flexibility of different segments of the device 4 has been selected to accommodate a particular vasculature passageway (where the curvature of the passageway may be determined in three dimensional view by, for example, MRI or CT).

FIG. 3 shows a side, fragmented, cross-sectional view of a catheter (or catheter guidewire) 30 showing various types of cuts which may be made generally transversely in the catheter for controlling flexibility thereof. In segment 30a of the catheter 30, cuts 34 are shown spaced closely together which would serve to increase flexibility of the catheter in that segment, whereas in segment 30b, cuts 38 are spaced farther apart but are made wider and this, likewise, serves to increase the flexibility of the catheter. Finally, in segment 30c of the catheter 30, cuts 42 are made deeper to thus increase flexibility. In other words, flexibility may be increased by (1) spacing the cuts closer together, (2) making the cuts wider, or (3) making the cuts deeper, i.e., controlling the beam.. Of course, all these techniques could be provided to achieve the desired flexibility.

The making of cuts in catheters and/or catheter guidewires to control flexibility is disclosed in co-pending United States patent application No. 08/819,611, filed March 17, 1997.

In addition to varying the flexibility of different segments of a catheter (or catheter guidewire) by selective use of cuts, selective annealing, abrading, varying wall or wire thickness, varying material properties of the catheter, etc. could also be employed.

It is to be understood that the above-described arrangements are only illustrative of the application of the principles of the present invention. Numerous modifications and alternative arrangements may be devised by those skilled in the art without departing from the spirit and scope of the present invention and the appended claims are intended to cover such modifications and arrangements.

## Claims

1. A catheter/guidewire device for threading into a vasculature passageway having a determinable pathway profile of curves and generally linear sections, said device comprising an elongate body having a distal end, a proximal end and intermediate sections, wherein selected intermediate sections are formed to be more flexible than other intermediate sections so that the selected sections coincide with the curves of the pathway profile when the device is threaded into the vasculature passageway.

2. A catheter/guidewire device as in Claim 1 wherein said selected intermediate sections are formed with a plurality of generally transverse cuts spaced-apart longitudinally along said selected intermediate sections.

3. A catheter/guidewire device as in Claim 2 wherein certain of said selected intermediate sections are formed to be more flexible than other of said selected intermediate sections, by spacing the cuts closer together.

4. A catheter/guidewire device as in Claim 2 wherein certain of said selected intermediate sections are formed to be more flexible than other of said selected intermediate sections, by forming the cuts to be deeper.

5. A catheter/guidewire device as in Claim 2 wherein certain of said selected intermediate sections are formed to be more flexible than other of said selected intermediate sections, by forming the cuts to be wider.

6. A catheter/guidewire device as in Claim 1 wherein certain of said selected intermediate sections are formed to be more flexible than other of said selected intermediate sections, so that said certain of said selected intermediate sections coincide with the curves of the pathway profile which have the greatest curvature, when the device is threaded into the vasculature passageway.

7. A catheter/guidewire device as in Claim 1 wherein at least some of the selected intermediate sections vary from one another in flexibility.

8. A catheter/guidewire device as in Claim 1 wherein the distal end of the wire is formed to be more flexible than other sections of the wire.

9. A catheter/guidewire device as in Claim 1 wherein at least one selected intermediate section is formed to be more flexible than the distal end.

10. A catheter/guidewire device as in Claim 1 wherein certain of said selected intermediate sections are formed to be more flexible than other of said selected intermediate sections, by annealing said certain sections.

11. A catheter/guidewire device as in Claim 1 wherein certain of said selected intermediate sections are formed to be more flexible than other of said selected intermediate sections, by abrading said certain sections.

12. A catheter/guidewire device as in Claim 1 wherein certain of said selected intermediate sections are formed to be more flexible than other of said selected intermediate sections, by reducing the device wall thickness at said certain sections.

13. A catheter/guidewire for threading into a vasculature passageway comprising an elongate body dimensioned for threading into the passageway, and including one or more sections intermediate the proximal end and the distal end, formed to be more flexible than other intermediate sections, some closer to the proximal end and some closer to the distal end.

14. A catheter/guidewire as in Claim 13 wherein said one or more sections are formed with a plurality of generally transverse cuts spaced-apart longitudinally along said one or more sections.

15. A catheter/guidewire as in Claim 14 wherein the longitudinal locations of said one ore more sections coincide generally with the longitudinal locations in the vasculature passageway having the greatest curvature.

16. A catheter/guidewire for threading into a vasculature passageway comprising an elongate body dimensioned for threading into the passageway, wherein the proximal end is more flexible than the distal end.
